(19) Europäisches Patentamt
European Patent Office
Office européen des brevets

(11) Veröffentlichungsnummer: **0 050 721**
**A1**

# (12) EUROPÄISCHE PATENTANMELDUNG

(21) Anmeldenummer: 81106802.2

(22) Anmeldetag: 01.09.81

(51) Int. Cl.³: C 07 D 307/60

(30) Priorität: 28.10.80 CH 8017/80
13.08.81 CH 5220/81

(43) Veröffentlichungstag der Anmeldung: 05.05.82 Patentblatt 82/18

(84) Benannte Vertragsstaaten: AT BE CH DE FR GB IT LI NL

(71) Anmelder: F. HOFFMANN-LA ROCHE & CO. Aktiengesellschaft

CH-4002 Basel(CH)

(72) Erfinder: Fizet, Christian, Dr.
18 rue de Bruebach
F-68440 Zimmersheim(FR)

(74) Vertreter: Cottong, Norbert A. et al,
Grenzacherstrasse 124 Postfach 3255
CH-4002 Basel(CH)

## (54) Verfahren zur Herstellung von Dihydro-4,4-dimethyl-2,3-furandion.

(57) Verfahren zur Herstellung von Dihydro-4,4-dimethyl-2,3-furandion durch Umsetzung eines Alkali- oder Erdalkalimetallsalzes der Dimethylbrenztraubensäure mit Formaldehyd in Gegenwart einer anorganischen Base.

EP 0 050 721 A1

Croydon Printing Company Ltd.

0050721



RAN 4224/050

F. Hoffmann-La Roche & Co. Aktiengesellschaft, Basel, Schweiz

Verfahren zur Herstellung von Dihydro-4,4-dimethyl-2,3-furandion.

Die vorliegende Erfindung betrifft ein neues Verfahren zur Herstellung von Dihydro-4,4-dimethyl-2,3-furandion der Formel

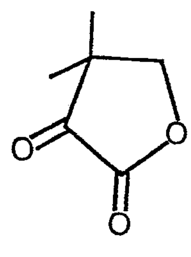

I

Diese Verbindung, welche auch als Ketopantolacton bezeichnet wird, stellt ein wichtiges Ausgangsmaterial für die Herstellung von optisch aktivem Dihydro-3-hydroxy-4,4-dimethyl-2(3H)-furanon (Pantolacton) dar.

Das Dihydro-4,4-dimethyl-2,3-furandion der Formel I kann z.B. durch Oxidation von racemischem Dihydro-3-hydroxy-4,4-dimethyl-2(3H)-furanon erhalten werden, wobei bisher jedoch unter Verwendung relativ teurer Oxidationsmittel wie Bleitetraacetat, N-Bromsuccinimid oder $CrO_3$ (Jones-Reagent) nur relativ geringe Ausbeuten erzielt wurden.

Cot/13.8.81

Eine Verbesserung dieses Verfahrens besteht in der Verwendung billigerer Oxidationsmittel wie feste Alkali- oder Erdalkalimetallhypochlorite in organischer Phase, wodurch zwar wesentlich höhere Ausbeuten erzielt werden, die Nachteile, unter oxidativen Bedingungen arbeiten zu müssen, jedoch nach wie vor vorhanden sind.

Es ist weiterhin bekannt, das Dihydro-4,4-dimethyl-2,3-furandion der Formel I direkt aus Dimethylbrenztraubensäure und Formaldehyd herzustellen. Dieses Verfahren hat jedoch den Nachteil, dass auch hier nur relativ geringe Ausbeuten erzielt werden, und dass das gewünschte Endprodukt auch nach Destillation in nicht ganz reiner Form anfällt.

Im Gegensatz zu letzterem Verfahren hat sich nun herausgestellt, dass man das gewünschte Dihydro-4,4-dimethyl-2,3-furandion der Formel I in wesentlich höheren Ausbeuten und in reiner Form erhält, wenn man statt reiner Dimethylbrenztraubensäure als Ausgangsmaterial ein Alkali- oder Erdalkalimetallsalz davon einsetzt.

Das erfindungsgemässe Verfahren ist demnach dadurch gekennzeichnet, dass man ein Alkali- oder Erdalkalimetallsalz der Dimethylbrenztraubensäure mit Formaldehyd in Gegenwart einer anorganischen Base umsetzt.

Als Alkali- oder Erdalkalimetallsalze der Dimethylbrenztraubensäure kommen, im Rahmen der vorliegenden Erfindung, insbesondere Natrium-, Kalium-, Magnesium- und Calciumsalze in Betracht. Bevorzugt ist das Natriumsalz.

Als anorganische Basen können im Rahmen der vorliegenden Erfindung insbesondere Hydroxide und Carbonate von Alkali- oder Erdalkalimetallen genannt werden, wie z.B. von Natrium, Kalium, Magnesium, Calcium und Barium. Bevorzugt ist das Kaliumcarbonat.

Die Umsetzung eines Alkali- oder Erdalkalimetallsalzes der Dimethylbrenztraubensäure mit Formaldehyd erfolgt zweckmässig in wässrigem Medium bei einer Temperatur von etwa 20°C bis etwa 50°C, vorzugsweise bei einer Temperatur von etwa 35°C bis etwa 45°C und insbesondere bei etwa 40°C. Um Nebenreaktionen, insbesondere die Bildung von unerwünschtem DL-Pantolacton zu verhindern, wird der Formaldehyd zweckmässig nicht auf einmal, sondern langsam und kontinuierlich dem Salz der Dimethylbrenztraubensäure zugefügt. Die Menge an vorhandener anorganischer Base ist nur insofern kritisch, als hierdurch ein pH-Wert des Reaktionsgemisches von etwa 11-13, vorzugsweise von etwa 12-12,5 erzielt werden soll. Bei Verwendung von schwächeren Basen, wie z.B. Kaliumcarbonat, soll sie jedoch wenigstens so gross sein, dass das Reaktionsgemisch heterogen bleibt; sie soll also zweckmässig im Ueberschuss vorhanden sein. Vorzugsweise beträgt die Menge an anorganischer Base in diesem Fall von etwa 1 Mol bis etwa 2 Mol, und insbesondere bis etwa 1,2 Mol bezogen auf das Dimethylbrenztraubensäuresalz. Bei Verwendung von stärkeren Basen, wie z.B. $Ba(OH)_2$, kann sie dem Reaktionsgemisch kontinuierlich zugesetzt werden um den gewünschten pH-Bereich aufrecht zu halten. Die Umsetzung kann sowohl ohne Inertgas als auch unter Inertgas durchgeführt werden. Bevorzugt wird unter Inertgas, wie z.B. unter Stickstoff, Argon und dergleichen, gearbeitet.

In den nachfolgenden Beispielen bedeutet der Ausdruck "Aether" jeweils Diäthyläther.

Beispiel 1

In einem 750 ml Vierhalskolben, versehen mit Rührer, Thermometer und Kühler, werden 42,8 g (0,31 Mol) Dimethylbrenztraubensäure-Natriumsalz in 40 ml Wasser suspendiert. Anschliessend werden portionsweise bei Raumtemperatur während ca. 2 Minuten 50 g (ca. 0,36 Mol) Kaliumcarbonat zugegeben. Es werden dann 18 ml 37%ige Formaldehyd-Lösung (0,24 Mol) konstant zugetropft (ca 9 ml pro Stunde) und dann während 70 Minuten nochmals 7 ml (0,093 Mol)[ca. 6 ml pro Stunde]. Hierauf wird das Reaktionsgemisch noch 60 Minuten bei 40$^{O}$C gerührt. Man gibt anschliessend 100 ml destilliertes Wasser zu und stellt die Reaktionslösung mit konzentrierter Salzsäure auf pH 1,2-1,3. Das Reaktionsgemisch wird dann noch eine halbe Stunde bei 40$^{O}$-50$^{O}$C gerührt und der pH mit konzentrierter Salzsäure bei 1,2-1,3 gehalten. Das Reaktionsgemisch wird dann über Nacht mit Aether extrahiert. Der Aether wird über Natriumsulfat getrocknet und am Rollverdampfer eingedampft. Der Rückstand - 39,5 g eines gelblichen Oeles - wird durch einen Kühler mit 35 ml Aether versetzt und geschüttelt, wobei Kristallisation eintritt und der Aether sich zum Rückfluss erhitzt. Nach dem Abkühlen (-5°C) werden die Kristalle abgenutscht und mit wenig kaltem Aether (-5°C) gewaschen. Man erhält 32,8 g Dihydro-4,4-dimethyl-2,3-furandion; Smp. 67,5°-68,5°C. Aus der Mutterlauge werden weitere 1,7 g gewonnen; Smp. 66,5°-68°C. Dies ergibt eine Totalausbeute von 34,5 g, d.h. ca. 87%.

Beispiel 2

In einem 500 ml Vierhalskolben, versehen mit Rührer, Thermometer, Kühler und Aufsatz für Inertgasbegasung, werden 35 g (0,253 Mol) Dimethylbrenztraubensäure-Natriumsalz in 32 ml destilliertem Wasser suspendiert. Anschliessend werden portionsweise bei Raumtemperatur während ca. 2 Minuten 31,2 g (0,29 Mol) wasserfreies Natriumcarbonat zugegeben.

Es werden dann bei 40°C während 2 Stunden 15 ml und danach während 1 Stunde noch 5,4 ml 35%-ige Formaldehydlösung (insgesamt 0,257 Mol) konstant zugetropft. Hierauf wird das Reaktionsgemisch noch 2 Stunden bei 40°C gerührt. Man gibt anschliessend 80 ml destilliertes Wasser zu und stellt die Reaktionslösung mit einer 50%-igen Schwefelsäurelösung auf pH 1,2-1,3. Das Reaktionsgemisch wird dann noch eine halbe Stunde bei 40°-50°C gerührt und der pH mit der Schwefelsäurelösung konstant gehalten. Das Reaktionsgemisch wird dann 12 Stunden mit Aether extrahiert. Der Aether wird über Natriumsulfat getrocknet und am Rollverdampfer eingedampft. Das zurückbleibende rohe Oel wird am Hochvakuum (0,4 mm Hg) bei 80°C von flüchtigen Anteilen befreit. Man erhält hierbei 29,2 g eines gelblichen Oeles. Dieses wird durch einen auf den Kolben aufgesetzten Kühler mit 30 ml Aether versetzt und geschüttelt wobei Kristallisation eintritt und der Aether sich zum Rückfluss erhitzt. Nach dem Abkühlen (-5°C) werden die Kristalle abgenutscht und mit wenig kaltem Aether (-5°C) gewaschen. Man erhält 21,8 g Dihydro-4,4-dimethyl-2,3-furandion; Smp. 67-68°C. Aus der Mutterlauge erhält man noch 1,5 g Kristalle mit einem Schmelzpunkt von 66-67°C. Dies ergibt eine Totalausbeute von 23,3 g, d.h. ca. 72%.

## Beispiel 3

In einem 500 ml Vierhalskolben, versehen mit Rührer, Thermometer, Kühler und Aufsatz für Argonbegasung, werden 35 g (0,253 Mol) Dimethylbrenztraubensäure-Natriumsalz in 32 ml destilliertem Wasser suspendiert. Unter Rühren wird der pH der Suspension mit Bariumhydroxid auf 12-12,5 gestellt und dann das Reaktionsgemisch auf 40°C erwärmt. Es werden dann während 2 Stunden 15 ml und danach während 1 Stunde weitere 5,4 ml 35%-ige Formaldehydlösung (insgesamt 0,257 Mol) konstant zugetropft. Gleichzeitig wird der pH der Lösung mit festem Bariumhydroxid bei pH 12-12,5 konstant gehalten. Man braucht insgesamt ca. 15 g $Ba(OH)_2$, $(8H_2O)$ (0,047 Mol). Das Reaktionsgemisch wird noch 1 Stunde

bei 40°C gerührt, wobei der pH weiterhin bei 12-12,5 gehalten wird. Man gibt anschliessend 80 ml destilliertes Wasser zu und stellt die Reaktionslösung mit einer 50%-igen Schwefelsäurelösung auf pH 1,2-1,3. Das Reaktionsgemisch wird dann noch eine halbe Stunde bei 40-50°C gerührt und der pH mit der Schwefelsäurelösung konstant gehalten. Nach Filtration wird das Reaktionsgemisch 12 Stunden mit Aether extrahiert. Der Aether wird über Natriumsulfat getrocknet und am Rollverdampfer eingedampft. Das erhaltene rohe Oel wird am Hochvakuum (0,4 mm Hg) bei 80°C von flüchtigen Anteilen befreit. Man erhält 29,1 g eines gelblichen Oeles. Dieses wird durch einen auf den Kolben aufgesetzten Kühler mit 30 ml Aether versetzt und geschüttelt, wobei Kristallisation eintritt und der Aether sich zum Rückfluss erhitzt. Nach dem Abkühlen (-5°C) werden die Kristalle abgenutscht und mit wenig kaltem Aether (-5°C) gewaschen. Man erhält 20,8 g Dihydro-4,4-dimethyl-2,3-furandion mit einem Schmelzpunkt von 67,5-68°C. Aus der Mutterlauge werden weitere 1,8 g Kristalle mit einem Schmelzpunkt von 66-68°C gewonnen. Dies ergibt eine Totalausbeute von 22,8 g, d.h. ca. 70,5%.

## Beispiel 4

In einem 350 ml Vierhalskolben, versehen mit Rührer, Thermometer, Kühler und Aufsatz für Argonbegasung, werden 25 g (0,0867 Mol) Dimethylbrenztraubensäure-Calciumsalz, 27,8 g (0,2 Mol) Kaliumcarbonat und 60 ml destilliertes Wasser gegeben. Dann werden bei 40°C während 2 Stunden 10 ml und danach während 1 Stunde noch 3,9 ml 35%-ige Formaldehydlösung (insgesamt 0,179 Mol) konstant zugetropft. Hierauf wird das Reaktionsgemisch noch 2 Stunden bei 40°C gerührt. Man gibt anschliessend 50 ml destilliertes Wasser zu und stellt die Reaktionslösung mit einer 50%-igen Schwefelsäurelösung auf pH 1,2-1,3. Das Reaktionsgemisch wird dann noch eine halbe Stunde bei 40-50°C gerührt und der pH mit der Schwefelsäurelösung konstant gehalten. Nach Filtration wird das Reaktionsgemisch 12 Stunden mit Aether extrahiert.

Der Aether wird über Natriumsulfat getrocknet und am Rollverdampfer eingedampft. Das erhaltene rohe Oel wird am Hochvakuum (0,4 mm Hg) bei 80°C von flüchtigen Anteilen befreit. Man erhält 21,3 g eines gelblichen Oeles. Dieses wird durch einen auf den Kolben aufgesetzten Kühler mit 25 ml Aether versetzt und geschüttelt, wobei Kristallisation eintritt und der Aether sich zum Rückfluss erhitzt. Nach dem Abkühlen (-5°C) werden die Kristalle abgenutscht und mit wenig kaltem Aether (-5°C) gewaschen. Man erhält 17,6 g Dihydro-4,4-dimethyl-2,3-furandion mit einem Schmelzpunkt von 67-68°C. Aus der Mutterlauge werden weitere 1,05 g Substanz gewonnen mit einem Schmelzpunkt von 66-67°C. Dies ergibt eine Totalausbeute von 18,65 g, d.h. ca. 84%.

Patentansprüche

1. Verfahren zur Herstellung von Dihydro-4,4-dimethyl-2,3-furandion, dadurch gekennzeichnet, dass man ein Alkali- oder Erdalkalimetallsalz der Dimethylbrenztraubensäure mit Formaldehyd in Gegenwart einer anorganischen Base umsetzt.

2. Verfahren nach Anspruch 1, dadurch gekennzeichnet, dass man die Umsetzung bei einem pH-Wert von etwa 11-13, insbesondere bei einem pH-Wert von etwa 12-12,5 durchführt.

3. Verfahren nach Anspruch 1 oder 2, dadurch gekennzeichnet, dass man als anorganische Base Kaliumcarbonat verwendet.

4. Verfahren nach Anspruch 3, dadurch gekennzeichnet, dass man die anorganische Base im Ueberschuss, bezogen auf das Dimethylbrenztraubensäuresalz einsetzt.

5. Verfahren nach Anspruch 4, dadurch gekennzeichnet, dass man die anorganische Base in einer Menge bis zu etwa 2 Mol, bezogen auf das Dimethylbrenztraubensäuresalz, einsetzt.

6. Verfahren nach Anspruch 5, dadurch gekennzeichnet, dass man die anorganische Base in einer Menge bis zu etwa 1,2 Mol, bezogen auf das Dimethylbrenztraubensäuresalz, einsetzt.

7. Verfahren nach einem der Ansprüche 1-6, dadurch gekennzeichnet, dass man als Alkalimetallsalz der Dimethylbrenztraubensäure das Natriumsalz als Ausgangsmaterial verwendet.

8. Verfahren nach einem der Ansprüche 1 bis 7, dadurch gekennzeichnet, dass man die Umsetzung bei einer Temperatur von etwa 30°C bis etwa 50°C durchführt.

9. Verfahren nach Anspruch 8, dadurch gekennzeichnet, dass man die Umsetzung bei einer Temperatur von etwa 35°C bis etwa 45°C durchführt.

10. Verfahren nach Anspruch 8 oder 9, dadurch gekennzeichnet, dass man die Umsetzung bei einer Temperatur von etwa 40°C durchführt.

***

0050721

Europäisches Patentamt

# EUROPÄISCHER RECHERCHENBERICHT

Nummer der Anmeldung
EP 81 10 6802.2

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| X | "Beilsteins Handbuch der organischen Chemie"<br>4. Auflage, Band 17, Teil 7<br>1975, SPRINGER VERLAG, Berlin, Heidelberg, New York<br>3. und 4. Ergänzungswerk, Seite 5848<br>-- | 1,3 | C 07 D 307/60 |
| A | Chemical Abstracts Band 43, Nr. 19<br>10. Oktober 1949<br>Columbus, Ohio, USA<br>S.H. LIPTON et al. "Synthesis of compounds related to pantothenic acid"<br>Spalte 7426e<br>& J. Am. Chem. Soc., Band 71, 1949<br>Seiten 2364 bis 2367<br>-- | | |
| A | Chemical Abstracts Band 65, Nr. 9<br>24. Oktober 1966<br>Columbus, Ohio, USA<br>C. BROQUET et al. "Condensation of 2-oxo-3,3-dimethyl-1,4-butanolide with benzene"<br>Spalte 13535g<br>& Compt. Rend., Ser. C, Band 262, Nr. 26<br>1966, Seiten 1891 bis 1893<br>-- | | |
| A | EP - A1 - 0 006 156 (F. HOFFMANN-LA ROCHE & CO.)<br>---- | | |

RECHERCHIERTE SACHGEBIETE (Int. Cl.³)

C 07 D 307/60

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 11-01-1982 | FROELICH |

EPA form 1503.1 06.78